# EUROPÄISCHE PATENTSCHRIFT

(11) **EP 1 427 822 B1**
(45) Veröffentlichungstag und Bekanntmachung des Hinweises auf die Patenterteilung: **16.07.2008**
(21) Anmeldenummer: 02777118.7
(22) Anmeldetag: 16.09.2002
(51) Int. Cl.: C12N 15/10

(54) **NUKLEINSÄURE FÜR EINEN KLONIERUNGSVEKTOR**
NUCLEIC ACID FOR A CLONING VECTOR
ACIDE NUCLEIQUE POUR UN VECTEUR DE CLONAGE

(30) Priorität: 16.09.2001 DE 10145553
(43) Veröffentlichungstag der Anmeldung: 16.06.2004
(73) Patentinhaber: Gene Architects AG, 68789 St. Leon-Rot (DE)
(72) Erfinder: HEIERMANN, Reinhard, Galway (IE)
(74) Vertreter: Isenbruck, Günter
(86) Internationale Anmeldenummer: PCT/EP2002/010375
(87) Internationale Veröffentlichungsnummer: WO 2003/025169

(56) Entgegenhaltungen:
- WO-A-01/79553
- US-A- 6 136 537
- ASSELBERGS F A M: "CREATION OF A NOVEL, VERSATILE MULTIPLE CLONING SITE CUT BY FOUR RARE-CUTTING HOMING ENDONUCLEASES" BIOTECHNIQUES, EATON PUBLISHING, NATICK, US, Bd. 20, April 1996 (1996-04), Seiten 558-562, XP002925648 ISSN: 0736-6205

## Beschreibung

Die Erfindung betrifft eine Nukleinsäure in einem oder geeignet zum Einbau in einen Klonierungsvektor, wobei die Nukleinsäure wenigstens eine für die Klonierung und/oder Expression benötigte funktionelle Nukleotidsequenz aufweist.

Klonierungsvektoren zeichnen sich dadurch aus, dass sie eine einzigartige Schnittstelle für eine Restriktionsendonuklease enthalten, und zwar in einer Region des Vektors, die weder für die Vermehrung des Vektors noch für das Überleben der Wirts-Zelle essentiell ist. Im Stand der Technik sind die ursprünglichen Klonierungsvektoren mit diesen Eigenschaften wie beispielsweise die Plasmide pBR322, pAT153 usw. fast vollständig durch Vektoren abgelöst worden, die Sequenzregionen mit einer Vielzahl aufeinanderfolgender Schnittstellen für verschiedene Restriktionsendonukleasen enthalten, sogenannte multiple Klonierungsstellen oder Polylinker. Häufig sind diese Kionierungsstellen/Polylinker zu polyfunktionellen Sequenzeinheiten ausgebaut, indem diese Sequenzregionen noch andere funktionelle Sequenzen enthalten, z.B. Promotorsequenzen für RNA-Polymerasen, die eine Transkription des klonierten Target-DNA-Fragments (d.h. des DNA-Fragments, das in den Vektor einkloniert wurde) ermöglichen, Terminationssequenzen und Polyadenylierungssignale, oder Sequenzen, die eine Aufreinigung des vom klonierten Target-DNA-Fragment exprimierten Proteins erleichtern.

Die herkömmlich zum Klonieren verwendeten Restriktionsendonukleasen des Typs II (im folgenden mit REII abgekürzt) haben die Eigenschaft, dass ihre spezifische DNA-Erkennungssequenz (i.d.R. 4-8 Basenpaare) gleichzeitig auch ihre spezifische DNA-Schnittstelle ist, und dass diese Erkennungssequenz punktsymmetrisch bezüglich ihres Zentrums, häufig auch ein Palindrom ist. Das heißt, wenn man beide Stränge betrachtet, ergibt sich in beiden Leserichtungen die gleiche Sequenz, mit der Folge, dass beim Schneiden der betreffenden DNA-Sequenz unabhängig von der Quelle der DNA stets die gleichen kompatiblen DNA-Enden entstehen.

Ein typisches Beispiel einer REII stellt EcoRI dar. Ihre Erkennungssequenz ist die 6er-Basenfolge ^{5'}GAATTC^{3'} auf beiden Strängen (also palindromisch), und ihre Schnittstelle liegt auf jedem Strang der doppelsträngigen DNA in 5'→3'-Richtung jeweils nach G und vor A :

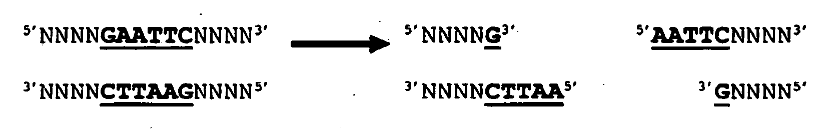
5'NNNNGAATTCNNNN3' 5'NNNNG3' 5'AATTCNNNN3'
3'NNNNCTTAAGNNNN5' 3'NNNNCTTAA5' 3'GNNNN5'

Beim Zusammenfügen von aufgeschnittenem Klonierungsvektor (große Buchstaben) und zu klonierendem Target-DNA-Fragment (kleine Buchstaben), die beide mit EcoRI erzeugt wurden, wird die Erkennungssequenz und Schnittstelle dieses Enzyms wieder regeneriert, und zwar an beiden Enden des betreffenden Target-DNA-Fragments.

5' NNNNG3'          AATTCNNNN3'   +Fragment   5' NNNNGaattcnnnnnnnngAATTCNNNN3'
3' NNNNCTTAA5'          3' GNNNN5'                3' NNNNCTTAAgnnnnnnnncttaaGNNNN5'

Dies gilt natürlich nicht nur für EcoRI, sondern für alle REII mit palindromischer Erkennungs- und Schnittsequenz.

Ein Nachteil der gängigen REII zeigt sich, wenn es darum geht, mehrere Target-DNA-Fragmente mit gleichen Enden in definierter Reihenfolge in einen Vektor zu klonieren. Da bei der Klonierung des ersten Target-DNA-Fragments die Erkennungssequenz und Schnittstelle der betreffendenden REII zweimal regeneriert wird, nämlich an beiden Enden des Target-DNA-Fragments, ist es nicht ohne weiteres möglich, in einem nachfolgenden Schritt nur eine der beiden Schnittstellen gezielt wieder zu öffnen, um ein zweites Target-DNA-Fragment einzufügen. In der Regel versucht der Fachmann in einem solchen Fall, einen Partialverdau durchzuführen. Dabei entstehen jedoch auch unerwünschte Schnittprodukte und damit später auch unerwünschte Klonierungsprodukte.

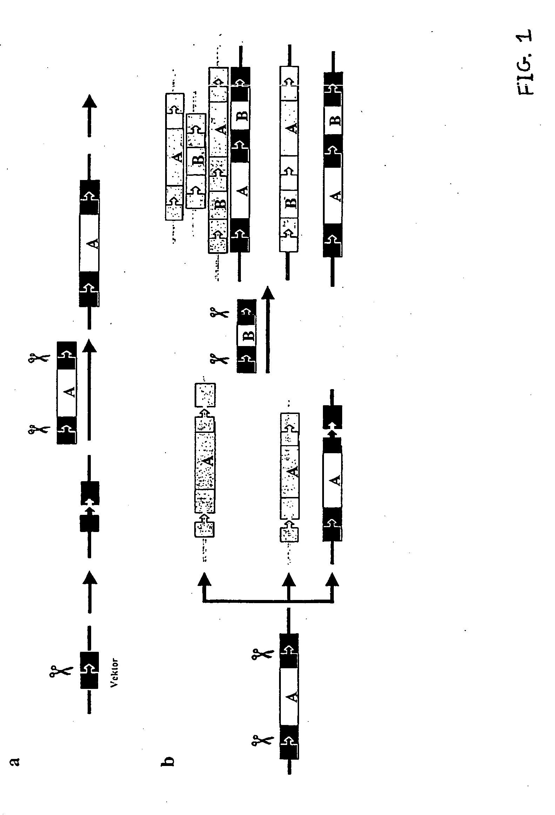

Das Prinzip dieser herkömmlichen Klonierungsverfahren mittels REII ist in Zeichnung 1 schematisch dargestellt, wobei die unerwünschten Klonierungsprodukte grau unterlegt sind.

Aus dieser Darstellung gemäß Zeichnung 1 ist ersichtlich, dass sich schon bei Einklonierung eines zweiten Target-DNA-Fragments die Suche nach dem gewünschten, richtigen Klonierungsprodukt zeit- und arbeitsaufwendig gestaltet. Bei drei, vier oder mehr einzuklonierenden Target-DNA-Fragmenten ist das herkömmliche Verfahren praktisch nicht mehr durchführbar, selbst wenn sich der Fachmann ergänzender Methoden wie zum Beispiel überlappender PCRs, Doppelverdau usw. bedient.

Im Stand der Technik ist noch eine weitere Gruppe von Restriktionsendonukleasen bekannt, allerdings in der Praxis sehr viel weniger verbreitet, nämlich die Restriktionsendonukleasen des Typs IIs (im folgenden abgekürzt mit REIIs). Diese Restriktionsendonukleasen unterscheiden sich von den REII zum einen dadurch, dass ihre Erkennungssequenz (i.d.R. 4-8 Basenpaare) gewöhnlich nicht symmetrisch und damit auch kein Palindrom ist, und zum anderen dadurch, dass Schnittstelle und Erkennungssequenz nicht notwendigerweise identisch sind, sondern häufig die Schnittstelle in einer definierten Entfernung zur Erkennungssequenz liegt. Es gibt folgende Möglichkeiten für die Position und Art der Schnittstelle:
**a.)** Beide DNA-Stränge werden in einem definierten Basenabstand außerhalb der Erkennungssequenz geschnitten ("outsite cutter"):
   Ein typisches und im Stand der Technik allgemein bekanntes Beispiel für einen solche "outsite cutter" ist die REIIs AarI. Ihre (seine) Erkennungssequenz ist die 7er-Basenfolge ^{5'}CACCTGC^{3'} auf dem einen DNA-Strang bzw. ^{5'}GCAGGTG^{3'} auf dem dazu komplementären DNA-Strang, und ihre Schnittstelle liegt zwischen der vierten und fünften beliebigen Base, die in 5'→3' Richtung auf die Erkennungssequenz ^{5'}CACCTGC^{3'} folgt bzw. zwischen der achten und neunten beliebigen Base, die in 5'→3' Richtung vor der Erkennungssequenz ^{5'}GCAGGTG^{3'} liegt: 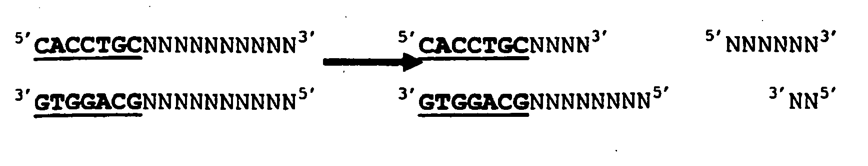
5' CACCTGCNNNNNNNNNN 3' 5' CACCTGCNNNN 3' 5' NNNNNN 3'

3' GTGGACGNNNNNNNNNN 5' 3' GTGGACGNNNNNNNN 5' 3' NN 5'
**b.)** Der Schnitt erfolgt auf einem Strang in definiertem Basenabstand außerhalb der Erkennungssequenz, auf dem anderen Strang an definierter Position innerhalb der Erkennungssequenz. ("partial outside cutter").
   Ein im Stand der Technik bekanntes Beispiel hierfür ist die REIIs BseNI: 
5'ACTGGNNNN3'    5'ACTGGN3'    5'NNN3'
3'TGACCNNNN5'    3'TGAC5'    3'CNNNN5'
**c.)** Beide Stränge werden an definierter Stelle innerhalb der Erkennungssequenz geschnitten.
   Ein im Stand der Technik bekanntes Beispiel hierfür ist die REIIs BssSI:

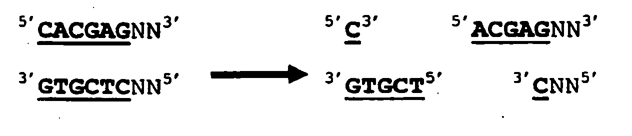
5'CACGAGNN3'          5'C3'          5'ACGAGNN3'

3'GTGCTCNN5'     3'GTGCT5'          3'CNN5'

Ohne zu den REIIs zu gehören, weist eins weitere Gruppe von Restriktionsendonukleasen ähnliche Eigenschaften wie REIIs auf, nämlich die "homing nucleases". Diese unterscheiden sich von den REIIs vor allem durch die (in der Regel wesentlich) längere Erkennungssequenz von gewöhnlich 8 - 20 Basenpaaren.

Ein typisches und im Stand der Technik allgemein bekanntes Beispiel für eine "homing nuclease" ist das Enzym I-HmuII ("outsite nicker") mit einer Erkennungssequenz von 24 Basen:

AGTAATGAGCCTAACGCTCAACAANNNNNNNNNNNNNNNNNNNNNNNNNNNNNNNNNNNNNNNNNNNNNNNNNNNNNNNNNNNN
TCATTACACGGATTGCGAGAAGTTNNNNNNNNNNNNNNNNNNNNNNNNNNNNNNNNNNNNNNNNNNNNNNNNNN N
N

Da "outside nicker" in der Regel nur einen Einzelstrangbruch an ihrer Schnittstelle erzeugen, können durch die Kombination von mindestens zwei solcher Enzyme neue vorteilhafte Schnittstellen, bei denen beide Nukleinsäurestränge durchtrennt werden, geschaffen werden.

Beim Schneiden einer DNA mit einem "outsite cutter" oder "outsite nicker" bleibt die Erkennungssequenz des betreffenden Enzyms auf einem der erzeugten DNA-Fragmente erhalten (während sie in dem Fall der analogen Verwendung einer REII beim Schneiden zerstört wird), und die beim Schneiden entstehenden Enden der erzeugten Fragmente sind in der Regel nicht kompatibel zu anderen, mit demselben Enzym erzeugten Fragmenten und infolgedessen nur direkt untereinander (direkt wechselseitig) wieder zusammenfügbar. Aus diesem Grund sind diese "outsite cutter"/"outsite nicker" für "konventionelle" Klonierungen ungeeignet, und andere Einsatzgebiete sind bisher kaum bekannt.

Eines der wenigen Beispiele für den Einsatz solcher "outsite cutter" ist im Katalog 2000/01 der Firma "Fermentas AB, Vilnius, Litauen" auf Seite 196/197 beschrieben. Dort werden "outsite cutter" zum Schneiden von Primer-Sequenzen in PCR-Produkten eingesetzt, um Enden für eine gerichtete Klonierung in konventionellen Vektoren zu erzeugen. Die nach dem Stand der Technik bekannten Verfahren zur gerichteten Synthese von Nukleinsäuremolekülen mittels REIIs weisen mehrere Nachteile auf. Viele der Verfahren benötigen den Einsatz weiterer, aufwendiger enzymatischer Reaktionen, wie z.B. Auffüllreaktionen mittels Polymerasen, Ausschneiden bzw. Reparatur bestimmter Nukleinsäurebereiche mittels Nukleasen oder die Modifizierung von Nukleinsäuren mittels dem Fachmann bekannter modifizierender Enzyme, wie z.B. Phosphatasen. Darüber hinaus gelingt es mit den bekannten Verfahren häufig nicht, mehrere Nukleinsäurefragmente gerichtet miteinander zu verknüpfen.

US 6,136,537 offenbart einen Klonierungsvektor, der an zwei vorbestimmten Stellen Erkennungssequenzen für Restriktionsenzyme des Typs IIs (REIIs) aufweist. Daran unmittelbar angrenzend befinden sich Schnittstellen für Restriktionsenzyme des Typs II (REII), die das Einbringen von Nukleinsäuren in den Vektor ermöglichen. Die Schnittstellen der REIIs liegen dabei innerhalb der REII Sequenz sind aber nicht mit dieser identisch. Ein Klonierungsvektor mit einer Einklonierungssequenz ("multiple cloning site") bestehend aus vier Mega- Nuklease Schnittstellen, die flankiert werden von Typ II Restriktionsenzym Schnittstellen, ist ebenfalls beschrieben worden (Asselbergs 1996, Biotechniques 20: 558-562).

Der vorliegenden Erfindung liegt die Aufgabe zugrunde, einen für die Klonierung und/oder Expression verwendbaren funktionellen Sequenzbereich bereitzustellen, bei dem die beschriebenen Nachteile beim Zusammenfügen mehrerer Target-DNA-Fragmente unter Verwendung von REII, sowie das nicht sequenzspezifische Schneiden von REIIs nicht auftreten. Eine weitere Aufgabe besteht darin, ein Verfahren zur Nukleinsäuresynthese bereitzustellen, bei dem aufwendige, enzymatische Reaktionen entfallen.

Die Aufgabe wird auch gelöst durch eine Nukleinsäure, die in einem Klonierungsvektor enthalten oder geeignet zum Einbau in einen Klonierungsvektor ist, die wenigstens eine für die Klonierung und/oder Expression benötigte funktionelle Nukleotidsequenz aufweist, dadurch gekennzeichnet, dass wenigstens eine funktionelle Nukleotidsequenz mit wenigstens einer REIIs-Erkennungssequenz oder "homing nuclease"-Erkennungssequenz derart gekoppelt ist, dass die Schnittstelle der betreffenden REIIs bzw. "homing nuclease" innerhalb der funktionellen Nukleotidsequenz liegt, wobei wenigstens eine funktionelle Nukleotidsequenz nach Schnitt mit der betreffenden REIIs bzw. "homing nuclease" inaktiv ist und die Schnittstelle der REIIs mit der Schnittstelle der REII identisch ist.

Diese Nukleinsäure ermöglicht es dem Fachmann, mindestens eine der vorhandenen Erkennungssequenzen ein und derselben REII und/oder mindestens eine der vorhandenen anderen funktionellen Sequenzen einzeln und gezielt mehrfach hintereinander anzusteuern. Als Nukleinsäure kommt insbesondere eine DNA oder eine RNA in Betracht.

Der Begriff "für die Klonierung und Expression benötigte funktionelle Nukleotidsequenz" steht hier insbesondere für jede beliebige vorhandene REII-Schnittstelle, daneben aber auch für RNA-Polymerase-Promotorsequenzen, für funktionelle Sequenzen der REIIs oder "homing nukleasen" selbst und für sonstige Sequenzen bzw. Teilsequenzen mit bestimmten und dem Fachmann bekannten Funktionen wie beispielsweise:
- Promotorsequenzen, Enhancer, Silencer, Terminationssequenzen, Polyadenylierungssequenzen (regulatorische Sequenzen)
- Gene, offene Leserahmen
- Protein- und RNP-Bindungsstellen
- Sequenzen, die eine Aufreinigung des vom klonierten Fragment exprimierten Proteins erleichtern

Der Begriff "gekoppelt" umfasst im vorliegenden Zusammenhang insbesondere die Bedeutungen,
- dass die Erkennungssequenz und die Schnittstelle der REIIs bzw. der "homing nuclease" innerhalb des für die Klonierung und/oder Expression benötigten funktionellen Sequenzbereichs liegen, d.h. Teilsequenz einer zum Einklonieren von Target-DNA-Fragmenten geeigneten oder sonstigen funktionellen Nukleotidsequenz sind,
   und
- dass die Schnittstelle innerhalb eines für die Klonierung und/oder Expression benötigten funktionellen Sequenzbereichs liegt, d.h. Teilsequenz einer zum Einklonieren von DNA-Fragmenten geeigneten oder sonstigen funktionellen Nukleotidsequenz ist, die Erkennungsequenz aber außerhalb des für die Klonierung und/oder Expression benötigten funktionellen Sequenzbereichs (innerhalb der Vektorsequenz) liegt.

Dabei ist es gegebenenfalls unerheblich, dass eine Erkennungssequenz oder Schnittstelle oder andere funktionelle Nukleotidsequenz zunächst fragmentiert vorliegt und erst für die Anwendung gezielt durch dem Fachmann geläufige Methoden zusammengeführt wird, wobei die erfindungsgemäße Nukleinsäure entsteht.

In einer bevorzugten Ausführungsvariante der erfindungsgemäßen Nukleinsäure sind insbesondere die Eigenschaften von REII und von REIIs zu Funktionseinheiten verbunden, welche jeweils gezielt angesteuert werden können. Jeder einzelnen, (u.U. in der betreffenden Nukleinsäure) auch mehrfach vorhandenen Erkennungssequenz einer beliebigen REII können eine oder mehrere beliebige REIIs zugeordnet werden, deren Erkennungssequenz - wie für REIIs üblich - während bzw. nach dem Restriktionsvorgang erhalten bleibt, und deren Schnittstelle innerhalb der betreffenden REII-Erkennungs- und Schnittsequenz liegt, und - falls gewünscht - mit der REII-Schnittstelle identisch ist. Als Folge davon kann diese REII-Schnittstelle durch die damit gekoppelte REIIs-Erkennungssequenz mit Hilfe des entsprechenden Enzyms (REIIs) gezielt und selektiv angesteuert, d. h. geöffnet werden, und - falls die Schnittstellen von REII und REIIs identisch sind, entstehen bei diesem Öffnen (Aufschneiden) die für die REII typischen, in der Regel kohäsiven DNA-Enden.

Diese bevorzugte Ausführungsform wird verwendet, um mehrere, gewünschte Nukleinsäurefragmente in beliebiger Reihenfolge seriell und linear zusammenzufügen, ohne dass dabei die Notwendigkeit weiterer enzymatischer Schritte besteht. Die Orientierung der inserierten Nukleinsäurefragmente lässt sich mit Methoden, die dem Fachmann bekannt sind, wie z.B. asymmetrische Restriktionsanalyse oder Sequenzierung, feststellen.

Die vorliegende Erfindung schließt demnach auch ein Verfahren zur seriellen, linearen Zusammenfügung von Nukleinsäurefragmenten unter Verwendung der erfindungsgemäßen Nukleinsäure ein, wobei das Verfahren die folgenden Schritte umfasst:
(i) Bereitstellung einer erfindungsgemäßen Nukleinsäure, bei der die Schnittstelle des REIIs bzw. der "homing nuclease" innerhalb einer REII-Erkennungs- und Schnittsequenz liegt und gegebenenfalls mit der REII-Schnittstelle identisch ist,
(ii) Öffnen der erfindungsgemäßen Nukleinsäure mit dem betreffenden REIIs oder REII,
(iii) Einfügen eines ersten Nukleinsäurefragments, das sowohl am 5'- als auch am 3'-Ende ein mit der REII der erfindungsgemäßen Nukleinsäure kompatibles Ende aufweist,
(iv) Öffnen der erfindungsgemäßen Nukleinsäure mit dem betreffenden REIIs (nicht REII), und
(v) Einfügen eines zweiten Nukleinsäurefragments, das sowohl am 5'- als auch am 3'-Ende ein mit der REII der erfindungsgemäßen Nukleinsäure kompatibles Ende aufweist,
wobei die Schritte (iv) bis (v) zum Einfügen weiterer Nukleinsäurefragmente beliebig oft wiederholt werden können.

Das erfindungsgemäße Verfahren ist in Beispiel 1 näher erläutert.

Erfindungsgemäß können in ein und dieselbe Nukleinsäure eine oder mehrere solcher REIIs-gekoppelten REII-Schnittstellen eingebaut sein, wobei in der Mehrzahl der Anwendungsfälle jede einzelne REIIs/REII-Kombination nur einmal in der Nukleinsäure vorkommt. Für spezielle Anwendung (gerichtete Klonierungen usw.) können aber auch Mehrfachkopplungen zweckmäßig sein, nämlich die Kopplung einer REIIs mit verschiedenen REII-Schnittstellen, bzw. die Kopplung verschiedener REIIs mit einer REII-Schnittstelle.

Gegenstand der Erfindung ist somit insbesondere eine Farnilie von funktionellen DNA-Klonierungs- und Expressions-Sequenzen mit jeweils unterschiedlichen Kombinationen aus REIIs-Erkennungssequenzen und REII-Schnittsequenzen.

Anstelle von, ergänzend zu oder in Kombination mit der Kopplung mit Erkennungssequenzen von REII können die REIIs/Homingnuklease-Erkennungssequenzen in gleicher Weise mit anderen funktionellen Teilsequenzen einer polyfunktionellen Sequenzeinheit (eines polyfunktionellen Polylinkers) gekoppelt sein, wobei "funktionelle Teilsequenz" jede beliebige funktionelle Sequenz (Promotor, Enhancer, Silencer, Protein-Bindungsstelle usw.) sein kann.

Die Erfindung ist diesbezüglich auch für die Kopplung von REIIs mit Promotorsequenzen von RNA-Polymerasen vorgesehen. Diese Promotorsequenzen von RNA-Polymerasen kommen, wenn überhaupt, dann üblicherweise ebenfalls in Mehrzahl in einem polyfunktionellen Polylinker vor, und in vielen Anwendungsfällen soll nur eine bestimmte für das gewünschte Vorhaben aktivierbar sein. Erfindungsgemäß ist es möglich, einzelne Promotorsequenzen gezielt durch Aufschneiden mit der jeweils damit gekoppelten REIIs bzw. "homing nuclease" zu inaktivieren, so dass nur eine gewünschte Promotorsequenz für den Einsatz der entsprechenden RNA-Polymerase funktionsfähig bleibt. Das hat unter anderem den Vorteil, dass von mehreren aneinandergereihten Target-DNA-Fragmenten (im Idealfall von einem kompletten Gen) sowohl sense- als auch antisense-Transkripte aus ein und demselben Konstrukt mit der gleichen RNA-Polymerase hergestellt werden können.

In diesem Zusammenhang beinhaltet die Erfindung ein Verfahren für die selektive Inaktivierung von Promotorsequenzen, wobei das Verfahren die folgenden Schritte umfasst:
(i) Bereitstellung einer erfindungsgemäßen Nukleinsäure, bei der
   (a) die Schnittstelle des REIIs bzw. der "homing nuclease" innerhalb einer Promotorsequenz liegt,
   (b) die Promotorsequenz zweimal in gegenläufiger Richtung vorliegt, wobei die beiden Promotorsequenzen identisch oder verschieden sein können und
   (c) beide Promotersequenzen mit mindestens je einem REIIs (REIIs-A, REIIs-B) gekoppelt sind, wobei REIIs-A von REIIs-B verschieden ist,
   und
(ii) Öffnen der Nukleinsäure mit wahlweise REIIs-A und/oder REIIs-B.

Das erfindungsgemäße Verfahren ist in Beispiel 3 näher erläutert. Zur Überprüfung einer erfolgreichen Inaktivierung einer Promotorsequenz stehen dem Fachmann zahlreiche Methoden wie z.B. In-vitro-Transkription zur Verfügung.

Bei der funktionellen Nukleotidsequenz kann es sich auch um die Erkennungssequenz einer REIIs oder einer "homing nuclease" handeln. Mittels einer solchen erfindungsgemäßen Nukleinsäure ist es dem Fachmann möglich, ganze Schaltsysteme zu konstruieren.

Die erfindungsgemäße Nukleinsäure, bei der es sich um eine DNA oder RNA handeln kann, kann nicht nur direkt (als Originärkonstrukt) sondern auch indirekt, beispielsweise als DNA mittels reverser Transkription aus einer RNA (als Transkriptionskonstrukt) hergestellt werden.

In einer weiteren bevorzugten Ausführungsform der vorliegenden Erfindung ist die erfindungsgemäße Nukleinsäure an ein Trägermaterial gekuppelt. Dies hat unter anderem den Vorteil, dass erhaltene Klonierungs- und/oder Expressionsprodukte direkt einer automatisierten Weiterverarbeitung oder anderen Analysen zugänglich sind. Die Erfindung umfasst alle Trägermaterialien, die geeignet sind, Nukleinsäuren daran zu koppeln. Nicht abschließend genannte Beispiele hierfür sind Agarose, Siliziumverbindungen, Polystyrenverbindungen, Teflon-Acrylamid, Polypropylen, Nylon, Sephacryl, Latex, paramagnetische Partikel, Nanopartikel und Zellulosederivate. Die Kopplung der Nukleinsäure erfolgt nach dem Fachmann bekannten Verfahren, sie kann sowohl kovalent als auch nicht-kovalent erfolgen. Es ist möglich, falls gewünscht, das Endprodukt auch wieder von dem Trägermaterial abzutrennen. Hierfür eignen sich - abhängig vom Trägermaterial - bestimmte Enzyme, chemische, hoch- und niedermolekulare Substanzen, sowie Einwirkung von Licht bestimmter Wellenlänge und Temperatureinwirkungen.

Gegenstand der vorliegenden Erfindung sind neben der beschriebenen Nukleinsäure auch Klonierungsvektoren, die eine solche Nukleinsäure enthalten, sowie Zellen, die einen oder mehrere solcher Klonierungsvektoren enthalten.

Es ist wünschenswert für die vorliegende Erfindung, dass der zum Einbau der erfindungsgemäßen Nukleinsäure verwendete Klonierungsvektor keine weiteren Erkennungs- bzw. Schnittstellen für REIIs bzw. "homing nucleases", die in der erfindungsgemäßen Nukleinsäure vorhanden sind, enthält.

Schließlich betrifft die vorliegende Erfindung ein Kit, mindestens enthaltend eine erfindungsgemäße Nukleinsäure, optional an ein Trägermaterial gekoppelt, mindestens ein geeignetes REIIs, optional in Kombination mit einem oder mehreren REII, eine Ligase, sowie gegebenenfalls andere geeignete Enzyme und geeignete Reaktionspuffer.

### Beschreibung der Zeichnungen

### Zeichnung 1:

### Klonierung von Nukleinsäurefragmenten mittels Typ II-Restriktionsendonukleasen

In Teil a ist die Klonierung eines Fragments A, in Teil b die zusätzliche Einfügung eines Fragments B beschrieben. Geschlossene Scheren repräsentieren eine beliebige Typ II-Restriktionsendonuklease, schwarze Kästchen mit Blockpfeil stellen die Erkennungs- und Schnittsequenz der REII dar. Kästchen A und B ist die zu klonierende Target-DNA.

### Zeichnung 2:

### Klonierung von Nukleinsäurefragmenten mittels Kombinationen von Typ II- und Typ IIs-Restriktionsendonukleasen

In Teil a ist die Klonierung eines Fragments B, in Teil b die zusätzliche Einfügung eines Fragments A beschrieben. Geschlossene Scheren repräsentieren eine beliebige Typ II-Restriktionsendonuklease, offene Scheren repräsentieren eine beliebige Typ IIs-Restriktionsendonuklease bzw. "homing nuclease". Kombinierte schraffierte/schwarze Kästchen mit Blockpfeil stellen die Erkennungssequenz der REIIs bzw. "homing nuclease" (schraffierte Kästchen), gekoppelt mit der Schnittsequenz einer beliebigen Typ II-Restriktionsendonuklease (schwarze Kästchen), dar. Kästchen A und B ist die zu klonierende Target-DNA.

### Zeichnung 3:

### REIIs/homing nuclease, gekoppelt mit funktionellen Sequenzen

Die Ziffern 1 und 2 bezeichnen die Erkennungssequenzen von TypIIs-Restriktionsendonukleasen, wobei 1 und 2 identisch oder verschieden sein können, A stellt eine funktionelle Sequenz (z.B. eine Promotorsequenz, s. Beispiel 3) dar, B ist eine insertierte Sequenz (z.B. eine Gen).

### Zeichnung 4:

### Mit REIIs gekoppelte, funktionelle Sequenz, in Kombination mit REIIs/REII-Kopplungen

Die Ziffern 1 und 2 bezeichnen die Erkennungssequenzen von TypIIs-Restriktionsendonukleasen, wobei 1 und 2 identisch oder verschieden sein können, A stellt eine funktionelle Sequenz (z.B. eine Promotorsequenz, s. Beispiel 3) dar, B kann ein Polylinker aus REIIs/"homing nuclease"-Erkennungssequenzen, gekoppelt mit REII-Schnittsequenzen sein.

### Zeichnung 5:

### Mehrere, mit REIIs gekoppelte, funktionelle Sequenzen, in Kombination mit mehreren REIIs/REII-Kopplungen

Die Ziffern 1 bis 5 bezeichnen Erkennungssequenzen von TypIIs-Restriktionsendonukleasen, wobei 1 bis 5 teilweise oder völlig identisch bzw. teilweise oder völlig verschieden sein können, A bis E sind beliebige funktionelle Sequenzen, a bis c sind verschiedene Polylinker.

### Zeichnung 6:

### Beispielhafte Struktur einer polyfunktionellen DNA-Klonierungsstelle (Polylinker) mit mehreren REIIs/"homing nuclease" steuerbaren REII-Schnittstellen

Die Erkennungssequenz jeder REIIs ist eingerahmt, die Erkennungs- und Schnittsequenz der damit gekoppelten REII ist grau unterlegt, die von den Einrahmungen ausgehenden Pfeile bezeichnen die Schnittstelle der REIIs.

Die Erfindung wird im folgenden anhand von Ausführungsbeispielen näher erläutert:

### Beispiel 1: Konstruktion einer Nukleinsäure in einem bzw. für einen Klonierungsvektor mit einer REIIs AarI gekoppelten EcoRI- (REII-) Schnittstelle.

Ausgangskomponenten sind ein beliebiges Plasmid, das wenigstens eine EcoRI-Schnittstelle enthält, und die REIIs AarI. Die REIIs Aarl ist bekanntermaßen dadurch charakterisiert, dass ihre Erkennungssequenz auf dem einen Strang ^{5'}**CACCTGC**^{3'} und auf dem dazu komplementären Strang dementsprechend ^{5'}**GCAGGTG**^{3'} lautet, und dass ihre Schnittstelle auf ersterem Strang zwischen der in 5'→3'-Richtung auf die Erkennungssequenz ^{5'}**CACCTGC**³ folgenden vierten und fünften Base liegt und auf dem dazu komplementären Strang in 5'→3'-Richtung zwischen der achten und neunten Base vor der Erkennungssequenz ^{5'}**GCAGGTG**^{3'} liegt.

AarI:

CACCTGCNNNNNNNNNN CACCTGCNNNN NNNNNN

GTGGACGNNNNNNNNNN GTGGACGNNNNNNNN NN

Auf dem Plasmid wird die EcoRI-Schnittstelle/Erkennungssequnez lokalisiert. Ausgehend von dieser EcoRI-Erkennungssequenz wird im Abstand von 3 Basen vor dieser Sequenz die Erkennungssequenz für die REIIs AarI eingefügt. Dieser Einbau erfolgt mit Methoden, die dem einschlägigen Fachmann bekannt und geläufig sind. Nach dem erfolgten Einbau sind die betreffende EcoRI- und die AarI-Schnittstelle überlagert, d.h. örtlich identisch. Infolge der Überlagerung kann die betreffende EcoRI-Schnittstelle jetzt sowohl mit der REII EcoRI, als auch mit der REIIs AarI gezielt geöffnet werden, wobei in beiden Fällen die für einen EcoRI-Schnitt typischen kohäsiven Enden entstehen:

AarI und EcoRI in der
erfindungsgemäßen Nukleinsäure Schnitt mit AarI oder EcoRI
AarI: AarI EcoRI AarI
CACCTGCNNNNNNNNNN → ACCTGCNNNGAATTCN → CACCTGCNNNG AATTCN
GTGGACGNNNNNNNNNN TGGACGNNNCTTAAGN GTGGACGNNNCTTAA GN

In diese geöffnete Schnittstelle wird jetzt ein Target-DNA-Fragment einkloniert, das die spezifischen EcoRI-Schnittenden aufweist und beispielsweise durch Schneiden mit EcoRI aus einer anderen DNA-Quelle gewonnen wurde. Dieses Target-EcoRI-Fragment ist im nachfolgenden Schema durch Kleinbuchstaben dargestellt:

AarI AarI EcoRI EcoRI

CACCTGCNNNG AATTCN CACCTGCNNNGaattcnnn---nnngAATTCN

GTGGACGNNNCTTAA GN → GTGGACGNNNCTTAAgnnn---nnncttaaGN

Um vor dieses EcoRI-Target-DNA-Fragment gezielt ein zweites EcoRI-Target-DNA-Fragment einzufügen, muss mit AarI geschnitten werden, denn nur der Einsatz dieser REIIs gewährleistet die gezielte Öffnung nur einer, nämlich der zu der AarI-Erkennungssequenz gehörigen (im Schema der linken) EcoRI-Schnittstelle,, während mit EcoRI beide vorhandenen Stellen geöffnet würden:

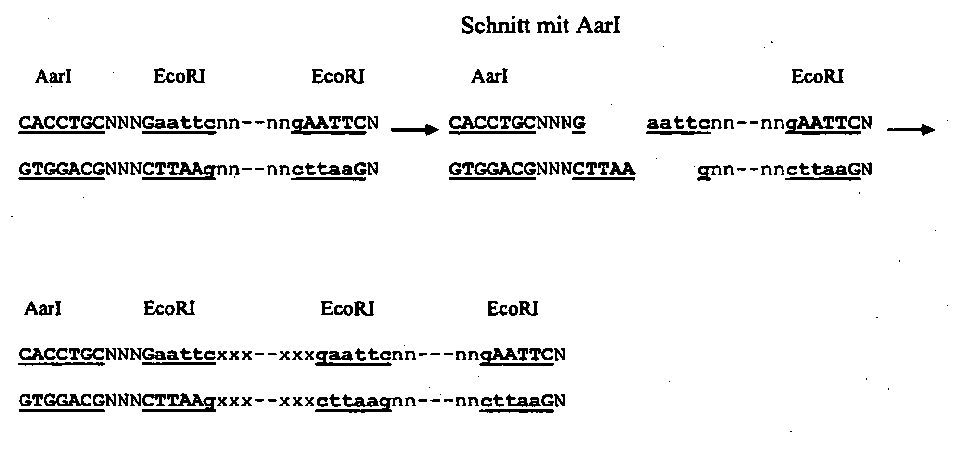
Schnitt mit AarI

AarI EcoRI EcoRI AarI EcoRI

CACCTGCNNNGaattcnn--nngAATTCN CACCTGCNNNG aattcnn--nngAATTCN

GTGGACGNNNCTTAAgnn--nncttaaGN GTGGACGNNNCTTAA gnn--nncttaaGN

AarI EcoRI EcoRI EcoRI

CACCTGCNNNGaattcxxx--xxxgaattcnn---nngAATTCN

GTGGACGNNNCTTAAgxxx--xxxcttaagnn---nncttaaGN

In derselben Weise können ein drittes, viertes und weitere EcoRI-Fragmente gezielt eingefügt werden und damit prinzipiell beliebig viele DNA-Fragmente mit EcoRI-Enden gezielt in gewünschter Reihenfolge in die Nukleinsäure bzw. den damit ausgestatteten Klonierungsvektor einkloniert und später exprimiert werden.

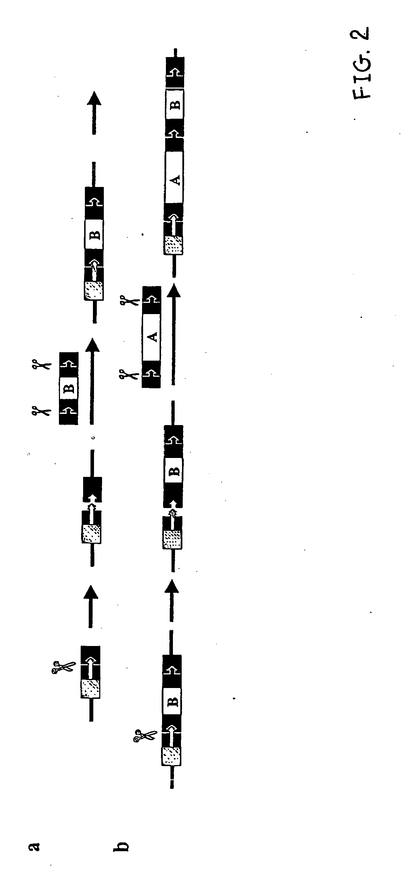

Anstelle der hier exemplarisch genannten Restriktionsendonukleasen EcoRI und AarI kann grundsätzlich jede beliebige Kombination einer REIIs und. REII eingesetzt werden. Eine schematische Darstellung des erfindungsgemäßen Konstruktionsprinzips ist in Zeichnung 2 wiedergegeben.

Der Vergleich der Darstellungen gemäß Zeichnung 1 und Zeichnung 2 zeigt unmittelbar den Vorteil der Erfindung, nämlich das Nichtauftreten unerwünschter Schnitt- und Klonierungsprodukte und damit die Ersparnis der zeitaufwendigen Suche nach dem gewünschten Produkt, insbesondere bei der Verknüpfung von mehr als zwei Target-DNA-Fragmenten.

### Beispiel 2: Struktur einer polyfunktionellen DNA-Klonierungsstelle (Polylinker) mit mehreren REIIs/"homing nuclease" steuerbaren REII-Schnittstellen

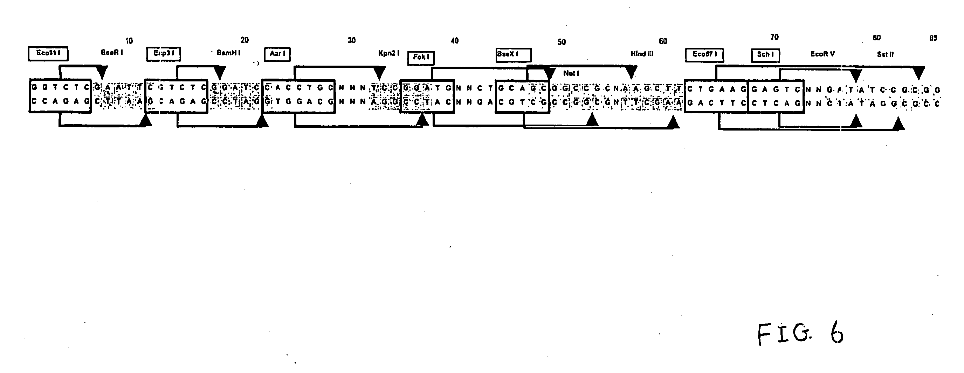

Die Struktur einer erfindungsgemäßen Nukleinsäure in der Ausführungsform als polyfunktionelle DNA-Klonierungsstelle (Polylinker) mit mehreren REIIs/"homing nuclease" steuerbaren REII-Schnittstellen ist in Zeichnung 6 schematisch dargestellt. Die Erkennungssequenz jeder REIIs ist eingerahmt, die Erkennungs- und Schnittsequenz der damit gekoppelten REII ist grau unterlegt, die von den Einrahmungen ausgehenden Pfeile bezeichnen die Schnittstelle der REIIs.

### Beispiel 3: Promotorsequenzen für eine RNA-Polymerase, die mit den REIIs AarI und Eco57I gezielt steuerbar ist.

Ausgangskomponenten sind ein Plasmid, in dem die Promotorsequenz für eine RNA-Polymerase, z.B. RNA-Polymerase T7, zweimal in gegenläufiger Richtung ausgebildet ist, sowie Erkennungssequenzen für die REIIs AarI und Eco57I. Die Eigenschaften von AarI sind im Beispiel 1 ausführlich beschrieben. Die REIIs Eco57I ist dadurch charakterisiert, dass ihre Erkennungssequenz auf dem einen Strang ^{5'}**CTGAAG**^{3'} und auf dem dazu komplementären Strang dementsprechend ^{5'}**CTTCAG**^{3'} lautet, und dass ihre Schnittstelle auf ersterem Strang in 5'→3'-Richtung zwischen der auf die Erkennungssequenz ^{5'}**CTGAAG**^{3'} folgenden sechszehnten und siebzehnten Base liegt und auf dem dazu komplementären Strang zwischen der vierzehnten und fünfzehnten Base in 5'→3'-Richtung vor der Erkennungssequenz ^{5'}**CTTCAG**^{3'} liegt.

AarI:

CACCTGCNNNNNNNNNN CACCTGCNNNN NNNNNN

GTGGACGNNNNNNNNNN GTGGACGNNNNNNNN NN

Eco57I:

CTGAAGNNNNNNNNNNNNNNNNNN → CTGAAGNNNNNNNNNNNNNNNN N

GACTTCNNNNNNNNNNNNNNNNNN GACTTCNNNNNNNNNNNNNN NNN

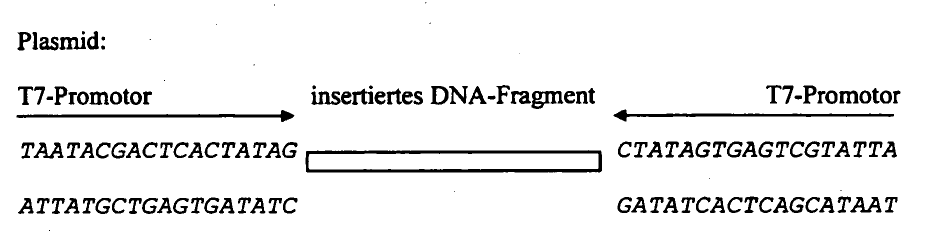
Plasmid:

T7-Promotor | insertiertes DNA-Fragment | T7-Promotor

TAATACGACTCACTATAG CTATAGTGAGTCGTATTA

ATTATGCTGAGTGATATC GATATCACTCAGCATAAT

Ausgehend von den beiden Promotorsequenzen werden in geeignetem Abstand die Erkennungssequenzen für die REIIs AarI und Eco57I mit dem Fachmann geläufigen Methoden eingefügt. Nach dem Einbau sind die Promotorsequenzen und die Restriktionsschnittstellen derart überlagert, dass gemäß nachfolgendem Schema der "linke" Promotor ausschließlich von AarI und der "rechte" Promotor ausschließlich von Eco57I steuerbar sind, indem das jeweilige Enzym die DNA in der betreffenden einen Promotorsequenz öffnet, dadurch die Promotoraktivität zerstört und nur noch eine Transkription von dem jeweils anderen Promotors zulässt:

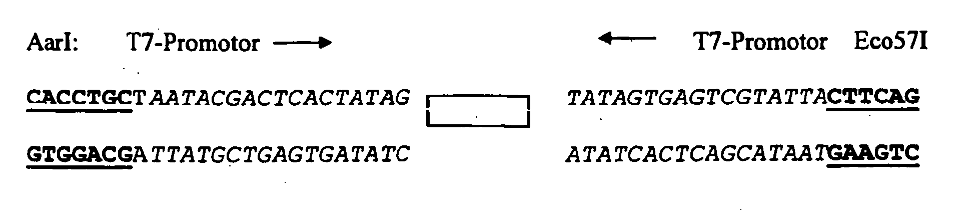
AarI: T7-Promotor ⟶ ⟵ T7-Promotor Eco57I
CACCTGCTAATACGACTCACTATAG TATAGTGAGTCGTATTACTTCAG
GTGGACGATTATGCTGAGTGATATC ATATCACTCAGCATAATGAAGTC

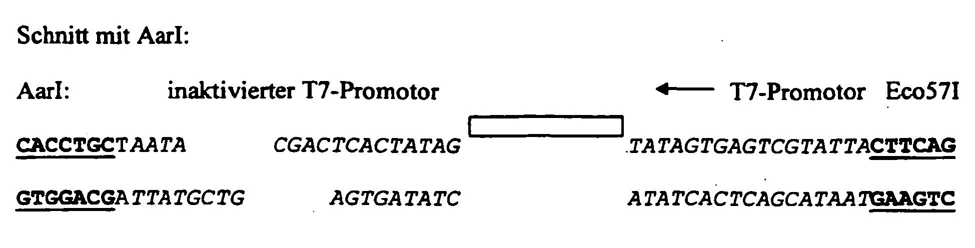
Schnitt mit AarI:

AarI: inaktivierter T7-Promotor T7-Promotor Eco57I

CACCTGCTAATA CGACTCACTATAG TATAGTGAGTCGTATTACTTCAG

GTGGACGATTATGCTG AGTGATATC ATATCACTCAGCATAATGAAGTC

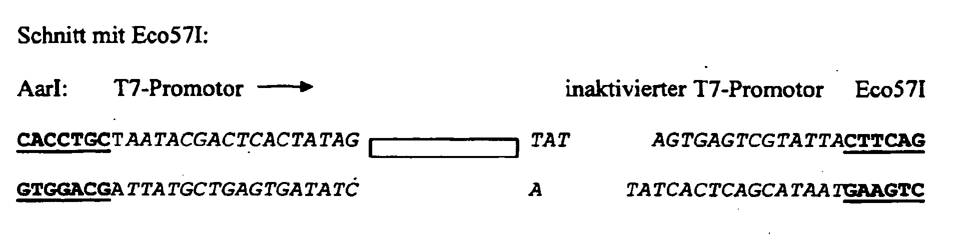
Schnitt mit Eco57I:

AarI: T7-Promotor → inaktivierter T7-Promotor Eco57I

CACCTGCTAATACGACTCACTATAG TAT AGTGAGTCGTATTACTTCAG

GTGGACGATTATGCTGAGTGATATC A TATCACTCAGCATAATGAAGTC

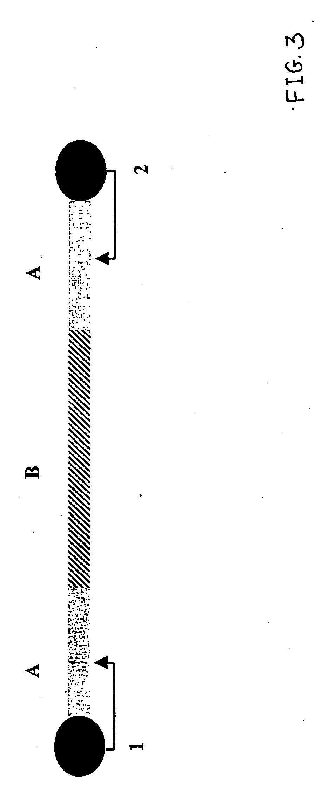

Verallgemeinert lässt sich diese erfindungsgemäße Kopplung einer REIIs/"homing nuclease" mit einer funktionellen DNA-Sequenz wie in Zeichnung 3 wiedergegeben darstellen.

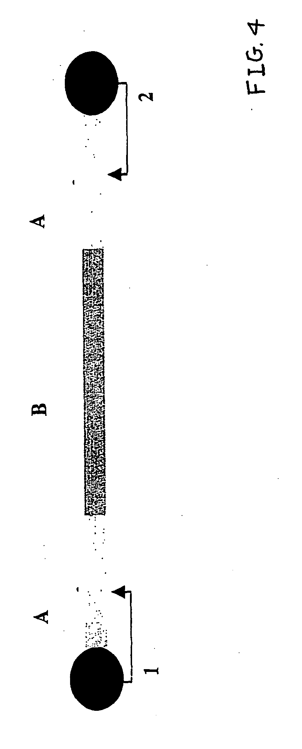

Beide Konstrukte, die Kopplung von REIIs/"homing nuclease" mit REII in Form einer einzelnen Einheit oder in Form einer polyfunktionellen DNA-Klonierungsstelle (eines Polylinkers) sowie die Kopplung von REIIs/"homing nuclease" mit anderen funktionellen Sequenzen können erfindungsgemäß selbstverständlich auch in einen einzigen Vektor verknüpft sein, wie das z.B. in Zeichnung 4 schematisch dargestellt ist.

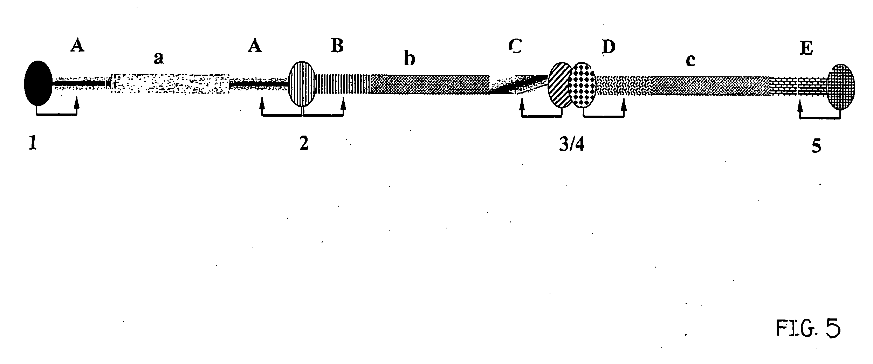

In entsprechender Weise sieht die Erfindung die Konstruktion von DNA-Klonierungs- und Expressions-Sequenzen vor, bei denen eine funktionelle Sequenz mehrfach oder verschiedene funktionelle Sequenzen mit verschiedenen REIIs/"homing nucleasen" gekoppelt sind, und bei denen diese REIIs/"homing nucleasen" gekoppelten funktionelle Sequenzen mit verschiedenen REIIs/"homing nuclease" gekoppelten REII-Schnittstellen in Verbindung stehen. Hierbei ist eine Vielzahl von Kombinationen gemäß dem in Zeichnung 5 dargestellten Schema denkbar und bei Bedarf konstruierbar.

## Patentansprüche

1. Nukleinsäure, die in einem Klonierungsvektor enthalten oder geeignet zum Einbau in einen Klonierungsvektor ist, die wenigstens eine für die Klonierung und/oder Expression benötigte funktionelle Nukleotidsequenz aufweist, **dadurch gekennzeichnet,**
**dass** wenigstens eine funktionelle Nukleotidsequenz mit wenigstens einer REIIs-Erkennungssequenz oder "homing nuclease"-Erkennungssequenz derart gekoppelt ist, dass die Schnittstelle der betreffenden REIIs bzw. "homing nuclease" innerhalb der funktionellen Nukleotidsequenz liegt, wobei wenigstens eine funktionelle Nukleotidsequenz die Erkennungssequenz und/oder Schnittstelle einer Restriktionsendonuklease-Typ II (REII) ist und die Schnittstelle der REIIs mit der Schnittstelle der REII identisch ist.

2. Nukleinsäure nach Anspruch 1, **dadurch gekennzeichnet,**
**dass** sie mehrere REIIs-gekoppelte REII-Schnittstellen aufweist.

3. Nukleinsäure nach Anspruch 1 oder 2, **dadurch gekennzeichnet,**
**dass** sie eine REIIs gekoppelt mit verschiedenen REII-Schnittstellen, oder mehrere verschiedener REIIs gekoppelt mit einer REII-Schnittstelle aufweist.

4. Nukleinsäure, die in einem Klonierungsvektor enthalten oder geeignet zum Einbau in einen Klonierungsvektor ist, die wenigstens eine für die Klonierung und/oder Expression benötigte funktionelle Nukleotidsequenz aufweist, **dadurch gekennzeichnet, dass** wenigstens eine funktionelle Nukleotidsequenz mit wenigstens einer REIIs-Erkennungssequenz oder "homing nuclease"-Erkennungssequenz derart gekoppelt ist, dass die Schnittstelle der betreffenden REIIs bzw. "homing nuclease" innerhalb der funktionellen Nukleotidsequenz liegt, wobei wenigstens eine funktionelle Nukleotidsequenz nach Schnitt mit der betreffenden REIIs bzw. "homing nuclease" inaktiv ist und die Schnittstelle der REIIs mit der Schnittstelle der REII identisch ist.

5. Nukleinsäure nach Anspruch 4, **dadurch gekennzeichnet,**
**dass** wenigstens eine funktionelle Nukleotidsequenz die Erkennungssequenz einer REIIs oder einer "homing nuclease" ist.

6. Nukleinsäure nach Anspruch 4, **dadurch gekennzeichnet,**
**dass** wenigstens eine funktionelle Nukleotidsequenz die Promotorsequenz einer RNA-Polymerase ist.

7. Nukleinsäure nach Anspruch 4, **dadurch gekennzeichnet,**
**dass** wenigstens eine funktionelle Nukleotidsequenz die Erkennungssequenz eines DNA-bindenden Proteins oder RNP-Partikels ist.

8. Nukleinsäure nach Anspruch 4, **dadurch gekennzeichnet,**
**dass** wenigstens eine funktionelle Nukleotidsequenz eine Enhancer-Sequenz ist.

9. Nukleinsäure nach Anspruch 4, **dadurch gekennzeichnet,**
**dass** wenigstens eine funktionelle Nukleotidsequenz eine Silencer-Sequenz ist.

10. Nukleinsäure nach Anspruch 4, **dadurch gekennzeichnet,**
**dass** wenigstens eine funktionelle Nukleotidsequenz eine Terminations-Sequenz ist.

11. Nukleinsäure nach Anspruch 4, **dadurch gekennzeichnet,**
**dass** wenigstens eine funktionelle Nukleotidsepquenz eine Polyadenylierungs-Sequenz ist.

12. Nukleinsäure nach einem der Ansprüche 1 bis 11, wobei die wenigstens eine funktionelle Nukleotidsequenz eine Sequenz ist, die eine Aufreinigung des vom klonierten Fragment exprimierten Proteins erleichtert.

13. Nukleinsäure nach einem der Ansprüche 1 bis 12, **dadurch gekennzeichnet,**
**dass** sie Bestandteil eines Klonierungsvektors ist.

14. Nukleinsäure nach einem der Ansprüche 1 bis 13, **dadurch gekennzeichnet, dass** sie an ein Trägermaterial gekoppelt ist.

15. Nukleinsäure nach Anspruch 14, wobei das Trägermaterial ausgewählt ist aus der Gruppe bestehend aus Agarose, Siliziumverbindungen, Polystyrenverbindungen, Teflon-Acrylamid, Polypropylen, Nylon, Sephacryl, Latex, paramagnetische Partikel, Nanopartikel und Zellulosederivate.

16. Verfahren zur seriellen, linearen Zusammenfügung von Nukleinsäurefragmenten unter Verwendung der Nukleinsäure gemäß einem der Ansprüche 1-3, das Verfahren umfassend die folgenden Schritte:
(i) Bereitstellung einer Nukleinsäure gemäß Ansprüchen 1 bis 3, bei der die Schnittstelle des REIIs bzw. der "homing nuclease" innerhalb einer REII-Erkennungs- und Schnittsequenz liegt und mit der REII-Schnittstelle identisch ist,
(ii) Öffnen der Nukleinsäure mit dem betreffenden REIIs oder REII,
(iii) Einfügen eines ersten Nukleinsäurefragments, das sowohl am 5'- als auch am 3'-Ende ein mit der REII der Nukleinsäure gemäß einem der Ansprüche 1-3 kompatibles Ende aufweist,
(iv) Öffnen der Nukleinsäure mit dem betreffenden REIIs (nicht REII), und
(v) Einfügen eines zweiten Nukleinsäurefragments, das sowohl am 5'- als auch am 3'-Ende ein mit der REII der Nukleinsäure gemäß einem der Ansprüche 1-3 kompatibles Ende aufweist,
wobei die Schritte (iv) bis (v) zum Einfügen weiterer Nukleinsäurefragmente beliebig oft wiederholt werden können.

17. Verfahren für die selektive Inaktivierung von Promotorsequenzen, das Verfahren umfassend die folgenden Schritte:
(i) Bereitstellung einer Nukleinsäure gemäß Anspruch 6, bei der
(a) die Schnittstelle des REIIs bzw. der "homing nuclease" innerhalb einer Promotorsequenz liegt,
(b) die Promotorsequenz zweimal in gegenläufiger Richtung vorliegt, wobei die beiden Promotorsequenzen identisch oder verschieden sein können und
(c) beide Promotersequenzen mit mindestens je einem REIIs (REIIs-A, REIIs-B) gekoppelt sind, wobei REIIs-A von REIIs-B verschieden ist.
und
(ii) Öffnen der Nukleinsäure mit wahlweise REIIs-A und/oder REIIs-B.

18. Klonierungsvektor, der eine Nukleinsäure nach einem der Ansprüche 1 bis 15 enthält.

19. Klonierungsvektor nach Anspruch 18, **dadurch gekennzeichnet, dass** er keine weiteren Erkennungs- bzw. Schnittstellen für die in besagter Nukleinsäure vorkommenden REIIs bzw. "homing nuclease" enthält.

20. Zellen, die einen Klonierungsvektor nach Anspruch 18 oder 19 bzw. eine Nukleinsäure nach einem der Ansprüche 1 bis 15 enthalten.

21. Kit, mindestens enthaltend eine Nukleinsäure nach einem der Ansprüche 1 bis 15, mindestens ein geeignetes REIIs, optional in Kombination mit einem oder mehreren REII, optional eine Ligase, sowie gegebenenfalls weitere geeignete Enzyme und geeignete Reaktionspuffer.

## Claims

1. Nucleic acid which is contained in a cloning vector or suitable for incorporation into a cloning vector, which has at least one functional nucleotide sequence needed for cloning and/or expression, **characterised in that**
at least one functional nucleotide sequence is coupled with at least one REIIs recognition sequence or "homing nuclease" recognition sequence such that the cutting site of the respective REIIs or "homing nuclease" lies within the functional nucleotide sequence, wherein at least one functional nucleotide sequence is the recognition sequence and/or cutting site of a restriction endonuclease type II (REII) and the cutting site of the REIIs is identical to the cutting site of the REII.

2. Nucleic acid according to claim 1, **characterised in that** it has several REIIs-coupled REII cutting sites.

3. Nucleic acid according to claim 1 or 2, **characterised in that**
it has an REIIs coupled with different REII cutting sites, or several different REIIs coupled with one REII cutting site.

4. Nucleic acid which is contained in a cloning vector or suitable for incorporation into a cloning vector, which has at least one functional nucleotide sequence needed for cloning and/or expression, **characterised in that** at least one functional nucleotide sequence is coupled with at least one REIIs recognition sequence or "homing nuclease" recognition sequence such that the cutting site of the respective REIIs or "homing nuclease" lies within the functional nucleotide sequence, wherein at least one functional nucleotide sequence is inactive after cutting with the respective REIIs or "homing nuclease" and the cutting site of the REIIs is identical to the cutting site of the REII.

5. Nucleic acid according to claim 4, **characterised in that** at least one functional nucleotide sequence is the recognition sequence of an REIIs or a "homing nuclease".

6. Nucleic acid according to claim 4, **characterised in that** at least one functional nucleotide sequence is the promoter sequence of an RNA polymerase.

7. Nucleic acid according to claim 4, **characterised in that** at least one functional nucleotide sequence is the recognition sequence of a DNA-binding protein or RNP particle.

8. Nucleic acid according to claim 4, **characterised in that** at least one functional nucleotide sequence is an enhancer sequence.

9. Nucleic acid according to claim 4, **characterised in that** at least one functional nucleotide sequence is a silencer sequence.

10. Nucleic acid according to claim 4, **characterised in that** at least one functional nucleotide sequence is a termination sequence.

11. Nucleic acid according to claim 4, **characterised in that** at least one functional nucleotide sequence is a polyadenylation sequence.

12. Nucleic acid according to one of claims 1 to 11, wherein the at least one functional nucleotide sequence is a sequence which facilitates purification of the protein expressed from the cloned fragment.

13. Nucleic acid according to one of claims 1 to 12, **characterised in that** it is a component of a cloning vector.

14. Nucleic acid according to one of claims 1 to 13, **characterised in that** is coupled to a carrier material.

15. Nucleic acid according to claim 14, wherein the carrier material is selected from the group consisting of agarose, silicon compounds, polystyrene compounds, Teflon acrylamide, polypropylene, nylon, sephacryl, latex, paramagnetic particles, nanoparticles and cellulose derivatives.

16. Method for the serial linear joining of nucleic acid fragments using the nucleic acid according to one of claims 1 - 3, the method comprising the following steps:
(i) providing a nucleic acid according to claims 1 to 3 in which the cutting site of the REIIs or the "homing nuclease" lies within an REII recognition and cutting sequence and is identical to the REII cutting site,
(ii)opening the nucleic acid with the respective REIIs or REII,
(iii) inserting a first nucleic acid fragment which has, both at the 5' and at the 3' end, an end compatible with the REII of the nucleic acid according to one of claims 1 - 3,
(iv)opening the nucleic acid with the respective REIIs (not REII), and
(v) inserting a second nucleic acid fragment which has, both at the 5' and at the 3' end, an end compatible with the REII of the nucleic acid according to one of claims 1 - 3,
(vi)
wherein steps (iv) to (v) can optionally often be repeated to insert other nucleic acid fragments.

17. Method for the selective inactivation of promoter sequences, the method comprising the following steps:
(i) providing a nucleic acid according to claim 6 in which
(a) the cutting site of the REIIs or the "homing nuclease" lies within a promoter sequence,
(b) the promoter sequence is present twice in the opposite direction, wherein both promoter sequences can be identical or different and
(c) both promoter sequences are coupled with at least one of each REIIs (REIIs-A, REIIs-B), wherein REIIs-A is different from REIIs-B
and
(ii)opening the nucleic acid with optionally REIIs-A and/or REIIs-B.

18. Cloning vector which contains a nucleic acid according to one of claims 1 to 15.

19. Cloning vector according to claim 18, **characterised in that** it contains no other recognition or cutting sites for the REIIs or "homing nuclease" occurring in said nucleic acid.

20. Cells which contain a cloning vector according to claim 18 or 19 or a nucleic acid according to one of claims 1 to 15.

21. Kit containing at least a nucleic acid according to one of claims 1 to 15, at least a suitable REIIs, optionally in combination with one or more REII, optionally a ligase, and optionally other suitable enzymes and suitable reaction buffers.

## Revendications

1. Acide nucléique, qui est contenu dans un vecteur de clonage ou est approprié à l'intégration dans un vecteur de clonage, qui présente au moins une séquence nucléotidique fonctionnelle nécessaire au clonage et/ou à l'expression, **caractérisé en ce que** au moins une séquence nucléotidique fonctionnelle est couplée à au moins une séquence d'identification de la REIIs ou une séquence d'identification « homing nuclease » de telle sorte que le site de clivage de la REIIs concernée ou « homing nuclease » se situe à l'intérieur de la séquence nucléotidique fonctionnelle, où au moins une séquence nucléotidique fonctionnelle est la séquence d'identification et/ou le site de clivage d'une endonucléase de restriction de type II (REII) et le site de clivage de la REIIs est identique au site de clivage de la REII.

2. Acide nucléique selon la revendication 1, **caractérisé en ce qu'**il présente plusieurs sites de clivage de la REII couplés à la REIIs.

3. Acide nucléique selon la revendication 1 ou 2, **caractérisé en ce qu'**il présente une REIIs couplée à divers sites de clivage de la REII ou plusieurs différentes REIIs couplées à un site de clivage de la REII.

4. Acide nucléique, qui est contenu dans un vecteur de clonage ou est approprié à l'intégration dans un vecteur de clonage, qui présente au moins une séquence nucléotidique fonctionnelle nécessaire au clonage et/ou à l'expression, **caractérisé en ce que** au moins une séquence nucléotidique fonctionnelle est couplée à au moins une séquence d'identification de la REIIs ou une séquence d'identification « homing nuclease » de telle sorte que le site de clivage de la REIIs concernée ou « homing nuclease » se situe à l'intérieur de la séquence nucléotidique fonctionnelle, où au moins une séquence nucléotidique fonctionnelle est inactive après clivage avec la REIIs concernée ou « homing nuclease » et où le site de clivage de la REIIs est identique au site de clivage de la REII.

5. Acide nucléique selon la revendication 4, **caractérisé en ce qu'**au moins une séquence nucléotidique fonctionnelle est la séquence d'identification d'une REIIs ou d'une « homing nuclease ».

6. Acide nucléique selon la revendication 4, **caractérisé en ce qu'**au moins une séquence nucléotidique fonctionnelle est la séquence promoteur d'une ARN-polymérase.

7. Acide nucléique selon la revendication 4, **caractérisé en ce qu'**au moins une séquence nucléotidique fonctionnelle est la séquence d'identification d'une protéine liant l'ADN ou d'une particule RNP.

8. Acide nucléique selon la revendication 4, **caractérisé en ce qu'**au moins une séquence nucléotidique fonctionnelle est une séquence activateur.

9. Acide nucléique selon la revendication 4, **caractérisé en ce qu'**au moins une séquence nucléotidique fonctionnelle est une séquence silenceur.

10. Acide nucléique selon la revendication 4, **caractérisé en ce qu'**au moins une séquence nucléotidique fonctionnelle est une séquence de terminaison.

11. Acide nucléique selon la revendication 4, **caractérisé en ce qu'**au moins une séquence nucléotidique fonctionnelle est une séquence de polyadénylation.

12. Acide nucléique selon l'une des revendications 1 à 11, où la au moins une séquence nucléotidique fonctionnelle est une séquence qui facilite la purification de la protéine exprimée par le fragment cloné.

13. Acide nucléique selon l'une des revendications 1 à 12, **caractérisé en ce qu'**il fait partie d'un vecteur de clonage.

14. Acide nucléique selon l'une des revendications 1 à 13, **caractérisé en ce qu'**il est couplé à un matériau support.

15. Acide nucléique selon la revendication 14, où le matériau support est choisi dans le groupe constitué de l'agarose, des composés de silicium, des composés de polystyrène, du Téflon-acrylamide, du polypropylène, du nylon, du Séphacryle, du latex, des particules paramagnétiques, des nanoparticules et des dérivés de cellulose.

16. Procédé pour l'assemblage linéaire en série de fragments d'acides nucléiques en utilisant l'acide nucléique selon l'une des revendications 1 à 3, le procédé comprenant les étapes suivantes :
(i) la mise à disposition d'un acide nucléique selon les revendications 1 à 3, dans lequel le site de clivage de la REIIs ou de la « homing nuclease » se situe à l'intérieur d'une séquence d'identification et de clivage de la REII et est identique au site de clivage de la REII,
(ii)l'ouverture de l'acide nucléique avec la REIIs ou la REII concernée,
(iii) l'insertion d'un premier fragment d'acide nucléique qui présente aussi bien sur l'extrémité 5' que sur l'extrémité 3' une extrémité compatible avec la REII de l'acide nucléique selon l'une des revendications 1 à 3,
(iv) l'ouverture de l'acide nucléique avec la REIIs concernée (et non REII), et
(v) l'insertion d'un deuxième fragment d'acide nucléique qui présente aussi bien sur l'extrémité 5' que sur l'extrémité 3' une extrémité compatible avec la REII de l'acide nucléique selon l'une des revendications 1 à 3,
où les étapes (iv) à (v) peuvent être répétées aussi souvent que voulu pour l'insertion d'autres fragments d'acide nucléique.

17. Procédé pour l'inactivation sélective de séquences promoteurs, le procédé comprenant les étapes suivantes :
(i) la mise à disposition d'un acide nucléique selon la revendication 6, dans lequel
(a) le site de clivage de la REIIs ou de la « homing nuclease » se situe à l'intérieur d'une séquence promoteur,
(b) la séquence promoteur se présente deux fois dans la direction antisens, où les deux séquences promoteurs peuvent être identiques ou différentes et
(c) les deux séquences promoteurs sont couplées à au moins respectivement une REIIs (REIIs-A, REIIs-B), où REIIs-A est différente de REIIs-B
et
(ii) l'ouverture de l'acide nucléique avec au choix la REIIs-A et/ou la REIIs-B.

18. Vecteur de clonage qui contient un acide nucléique selon l'une des revendications 1 à 15.

19. Vecteur de clonage selon la revendication 18, **caractérisé en ce qu'**il ne contient aucun autre site de clivage ou d'identification pour la REIIs ou « homing nuclease » apparaissant dans ledit acide nucléique.

20. Cellules qui contiennent un vecteur de clonage selon la revendication 18 ou 19 ou un acide nucléique selon l'une des revendications 1 à 15.

21. Kit, contenant au moins un acide nucléique selon l'une des revendications 1 à 15, au moins une REIIs appropriée, le cas échéant en combinaison avec une ou plusieurs REII, le cas échéant une ligase, ainsi que, le cas échéant, d'autres enzymes appropriées et tampons de réaction appropriés.


### SEQUENCE LISTING

<110> Gene Architects AG
<120> Nukleinsäure für einen Klonierungsvektor
<130> GA62022PC
<160> 6
<170> PatentIn version 3.1
<210> 1
   <211> 6
   <212> DNA
   <213> Escherichia coli
<220>
   <221> mise-feature
   <223> Restriction site Eco RI
<400> 1
   gaattc 6
<210> 2
   <211> 7
   <212> DNA
   <213> Arthrobacter aurescens
<220>
   <221> misc_feature
   <223> Restriction site Aar I
<400> 2
   cacctgc 7
<210> 3
   <211> 5
   <212> DNA
   <213> Bacillus species N
<220>
   <221> misc_feature
   <223> Restriction site BseNI
<400> 3
   actgg 5
<210> 4
   <211> 6
   <212> DNA
   <213> Bacillus stearothermophilus
<220>
   <221> misc_feature
   <223> Restriction site BsssI
<400> 4
   cacgag 6
<210> 5
   <211> 24
   <212> DNA
   <213> Bacillus subtilis
<220>
   <221> misc_feature
   <223> Restriction site I-HmuII
<400> 5
   tcattacacg gattgcgaga agtt 24
<210> 6
   <211> 6
   <212> DNA
   <213> Escherichia coli
<220>
   <221> misc_feature
   <223> Restriction site Eco57I
<400> 6
   ctgaag 6